# EUROPEAN PATENT APPLICATION

(11) **EP 3 398 506 A1**
(43) Date of publication of application: **07.11.2018**
(21) Application number: 17305491.7
(22) Date of filing: 02.05.2017
(51) Int. Cl.: A61B 5/00, A61B 5/05, A61B 5/024, A61B 5/0205, A61B 5/08, A61B 5/113

(54) **METHOD AND DEVICE FOR MONITORING PHYSIOLOGICAL SIGNAL CHARACTERISTICS OF A LIVING SUBJECT**

(71) Applicant: Thomson Licensing, 92130 Issy-les-Moulineaux (FR)
(72) Inventor: LOUZIR, Ali, 35576 Cesson-Sévigné Cedex (FR); KUMAR, Rupesh, 35576 Cesson-Sévigné Cedex (FR); LE NAOUR, Jean-Yves, 35576 Cesson-Sévigné Cedex (FR)
(74) Representative: Vidon Brevets & Stratégie

(57) **Abstract**

A method for monitoring physiological signal characteristics of a living subject is disclosed. A salient idea is to electromagnetically couple the antennas of respectively a networking device and a monitoring device, the networking device transmitting a wireless signal, the monitoring device receiving a first and a second signals from the electromagnetic coupling, the first and the second signals comprising a reflection of the transmitted signal on a living subject as well as a part of the transmitted signal resulting from the antenna coupling. A self-interference cancellation processing is applied to the received first and second signals for extracting the reflected signal from the first signal prior to amplifying and mixing with the received second signal so as to monitor physiological signal characteristics of the living subject.

The electromagnetic coupling is advantageous as it allows to pair the monitoring device, being for example a small size sensing module to any existing wireless network piece of equipment and to use existing network traffic as a source diagnostic signal. The self-interference cancelation is further advantageous as it allows to attenuate the large amplitude of the received first and second signals (being large in comparison with the reflected portion of the transmitted signal which is more attenuated through reflection and path losses) before amplifying and mixing so as to avoid a RF saturation of the receiver. This allows to guarantee good RF amplification conditions for the system reception.

## Description

### 1. TECHNICAL FIELD

The technical field of the disclosed method and device is related to physiological measurement techniques.

### 2. BACKGROUND ART

Several methods are known to help medical professionals to access and visualize physiological signal characteristics related to a moving organ such as the heart or the lungs. An electrocardiogram for example is known to obtain physiological signal characteristics related to the heartbeat. Most of the techniques of the medical professional domain rely on invasive material to be put on the patient. With the development of health care, there is a need for new methods and wearable-free solutions for wireless sensing of breathing and/or heartbeat of people in their normal live, without requiring them to go to a doctor or to a hospital.

US patent US 9035775B2 describes a Doppler based physiological monitoring device comprising a Wi-Fi module generating an IEEE 802.11 diagnostic signal. The differences between the source diagnostic signal and the return signal (i.e., the modified signal after undergoing modification as it passes through the patient) are then analyzed by the device to monitor heart and respiratory rates. Indeed, using Doppler Effect principles, heart rate and motion are measured from the differences in frequency, phase, and/or wavelength between the source signal and the modified signal reflected back from the heart and lungs moving within the patient.

**Figure 1** illustrates the monitoring device of US 9035775B2. The monitoring device 100 comprises a Wi-Fi transmitter 10 with a first antenna 11 for transmitting the diagnosis source signal *D.* The monitoring device further comprises a sensing module 101, embedded in the monitoring device 100, and electrically connected to an output of the Wi-Fi transmitter 10 via a RF splitter 17, for receiving the diagnosis source signal *D.* The sensing module 101 of the monitoring device 100 further comprises a second antenna 12 for receiving a return signal *r* (being a reflected signal after undergoing modification as it passes through the living subject 1). The second antenna 12 is decoupled from the first antenna 11 so as to not be polluted by the diagnosis source signal *D* being transmitted by the first antenna 11. The received return signal *r* is processed through a band pass filter 13 for attenuating unwanted frequencies and a LNA 14 (low noise amplifier) for amplifying the return signal *r* after it leaves the band pass filter 13. The filtered and amplified return signal rand the diagnosis source signal *D* (received from the RF splitter 17) are fed into a mixer 15, for obtaining a baseband signal. The obtained baseband signal is further processed by a processing module 18, for measuring and monitoring heart rate and lung motion, using Doppler Effect principles, from the differences in frequency, phase, and/or wavelength between the source diagnosis signal and the modified signal reflected back from the heart and lungs moving within the patient.

The monitoring device 100 of US 9035775B2 is a dedicated device generating its own dedicated Wi-Fi traffic interfering with any existing Wi-Fi devices a user may already have. Some new wearable-free breathing and/or heartbeat monitoring solutions are needed for operating in dense urban areas, where the level of Wi-Fi traffic is already important.

### 3. SUMMARY

A salient idea is to electromagnetically couple the antennas of respectively a networking device and a monitoring device, the networking device transmitting a wireless signal, the monitoring device receiving a first and a second signals from the electromagnetic coupling, the first and the second signals comprising a reflection of the transmitted signal on a living subject as well as a part of the transmitted signal resulting from the antenna coupling. A self-interference cancellation processing is applied to the received first and second signals for extracting the reflected signal from the first signal prior to amplifying and mixing with the received second signal so as to monitor physiological signal characteristics of the living subject.

The electromagnetic coupling is advantageous as it allows to pair the monitoring device, being for example a small size sensing module to any existing wireless network piece of equipment and to use existing network traffic as a source diagnostic signal. The self-interference cancelation is further advantageous as it allows to attenuate the large amplitude of the received first and second signals (being large in comparison with the reflected portion of the transmitted signal which is more attenuated through reflection and path losses) before amplifying and mixing so as to avoid a RF saturation of the receiver. This allows to guarantee good RF amplification conditions for the system reception.

To that end, a method for monitoring physiological signal characteristics of a living subject is disclosed. The method comprises:
- receiving a first signal in a sensing module, the first signal comprising a reflected signal of a transmitted signal on the living subject, the transmitted signal being transmitted by a wireless transmitter;
- receiving a second signal in the sensing module coupled to the wireless transmitter;
- amplifying the received first signal;
- mixing the received second signal and the amplified received first signal to monitor the physiological signal characteristics;
the method being characterized in that:
- a receiver antenna of the sensing module is electromagnetically coupled to a transmitter antenna of the wireless transmitter;
- a self-interference cancellation is applied to the received first and second signals before amplifying and mixing.

According to a particularly advantageous variant, the received second signal is obtained from a RF coupler connected to the receiver antenna of the sensing module.

According to another particularly advantageous variant, applying a self-interference cancellation comprises applying a variable gain amplifier and a variable phase shifter to the received second signal, the variable gain amplifier operating with amplitude saturation, the variable gain amplifier and the variable phase shifter being configured to extract the reflected signal from the received first signal.

According to another particularly advantageous variant, applying a self-interference cancellation comprises limiting the received second signal for removing the reflected signal, and applying a variable gain amplifier and a variable phase shifter to the received limited second signal, the variable gain amplifier and the variable phase shifter being configured to extract the reflected signal from the received first signal.

According to another particularly advantageous variant, the variable gain amplifier and the variable phase shifter are statically configured.

According to another particularly advantageous variant, the variable gain amplifier and the variable phase shifter are dynamically controlled.

According to another particularly advantageous variant, the wireless transmitter is a Wi-Fi access point.

In a second aspect, a sensing device for monitoring physiological signal characteristics of a living subject is also disclosed. The sensing device comprises:
- a receiver antenna for receiving a first signal, the first signal comprising a reflected signal of a transmitted signal on the living subject, the transmitted signal being transmitted by a wireless transmitter;
- means for receiving a second signal from a coupling to the wireless transmitter;
- means for amplifying the received first signal;
- means for mixing the received second signal and the amplified received first signal to monitor the physiological signal characteristics;
the sensing device being characterized in that:
- the coupling is an electromagnetic coupling between the receiver antenna and a transmitter antenna of the wireless transmitter;
- the sensing device further comprises self-interference cancellation means for cancelling self-interferences between the received first and second signals before amplifying and mixing.

According to another particularly advantageous variant, the self-interference cancellation means comprise a variable gain amplifier and a variable phase shifter, the variable gain amplifier operating with amplitude saturation, the variable gain amplifier and the variable phase shifter being configured to extract the reflected signal from the received first signal.

According to another particularly advantageous variant, the variable gain amplifier and the variable phase shifter are dynamically controlled.

In a third aspect, a sensing device for monitoring physiological signal characteristics of a living subject is also disclosed. The sensing device comprises:
- a receiver antenna for receiving a first signal, the first signal comprising a reflected signal of a transmitted signal on the living subject, the transmitted signal being transmitted by a transmitter antenna of a wireless transmitter, the receiver antenna being adapted to be electromagnetically coupled to the transmitter antenna;
- a coupler connected to the receiver antenna for receiving a second signal;
- a self-interference cancellation circuitry configured to cancel self-interferences between the first and the second received signals, by attenuating the received first signal;
- an amplifier configured to amplify the received first signal after self-interference cancellation;
- a mixer configured to mix the received second signal and the amplified received first signal;
- a processor configured to monitor the physiological signal characteristics from the mixed signals.

According to another particularly advantageous variant, the self-interference cancellation circuitry comprises a variable gain amplifier and a variable phase shifter, the variable gain amplifier operating with amplitude saturation, the variable gain amplifier and the variable phase shifter being configured to extract the reflected signal from the received first signal.

According to another particularly advantageous variant, the processor is further configured to dynamically control the variable gain amplifier and the variable phase shifter.

In a fourth aspect, an apparatus for monitoring physiological signal characteristics of a living subject is also disclosed. The apparatus comprises a wireless transmitter adapted to transmit a transmitted signal with a transmitter antenna, the apparatus further comprising a sensing device, the sensing device comprising:
- a receiver antenna for receiving a first signal, the first signal comprising a reflected signal of the transmitted signal on the living subject;
- means for receiving a second signal from a coupling to the wireless transmitter;
- means for amplifying the received first signal;
- means for mixing the received second signal and the amplified received first signal to monitor the physiological signal characteristics;
the apparatus being characterized in that:
- the coupling being an electromagnetic coupling between the receiver antenna and a transmitter antenna of the wireless transmitter;
- the sensing device further comprises self-interference cancellation means for cancelling self-interferences between the received first and second signals before amplifying and mixing.

According to another particularly advantageous variant, the self-interference cancellation means comprise a variable gain amplifier and a variable phase shifter, the variable gain amplifier operating with amplitude saturation, the variable gain amplifier and the variable phase shifter being configured to extract the reflected signal from the received first signal.

According to another particularly advantageous variant, the variable gain amplifier and the variable phase shifter are statically configured.

In a fifth aspect, an apparatus for monitoring physiological signal characteristics of a living subject is also disclosed. The apparatus comprises a wireless transmitter adapted to transmit a transmitted signal with a transmitter antenna, the apparatus further comprising a sensing device, the sensing device comprising:
- a receiver antenna for receiving a first signal, the first signal comprising a reflected signal of a transmitted signal on the living subject, the transmitted signal being transmitted by a transmitter antenna of a wireless transmitter, the receiver antenna being adapted to be electromagnetically coupled to the transmitter antenna;
- a coupler connected to the receiver antenna for receiving a second signal;
- a self-interference cancellation circuitry configured to cancel self-interferences between the first and the second received signals, by attenuating the received first signal;
- an amplifier configured to amplify the received first signal after self-interference cancellation;
- a mixer configured to mix the received second signal and the amplified received first signal;
- a processor configured to monitor the physiological signal characteristics from the mixed signals.

According to another particularly advantageous variant, the self-interference cancellation circuitry comprises a variable gain amplifier and a variable phase shifter, the variable gain amplifier operating with amplitude saturation, the variable gain amplifier and the variable phase shifter being configured to extract the reflected signal from the received first signal.

According to another particularly advantageous variant, the variable gain amplifier and the variable phase shifter are statically configured.

According to another particularly advantageous variant, the processor is further configured to dynamically control the variable gain amplifier and the variable phase shifter.

In a sixth aspect, a computer program product for monitoring physiological signal characteristics of a living subject is also disclosed. The computer program product comprises program code instructions executable by a processor for:
- receiving a first signal from a receiver antenna, the first signal comprising a reflected signal of a transmitted signal on the living subject, the transmitted signal being transmitted by a transmitter antenna of a wireless transmitter, the receiver antenna being adapted to be electromagnetically coupled to the transmitter antenna;
- receiving a second signal from a coupling of the first received signal;
- controlling a self-interference cancellation circuitry for cancelling self-interferences between the first and second received signals prior to amplification;
- monitoring the physiological signal characteristics from a mixing of the received second signal and the amplified attenuated received first signal.

In a seventh aspect, a computer-readable storage medium storing computer-executable program instructions is also disclosed. The computer-readable storage medium comprises instructions of program code executable by at least one processor to:
- receive a first signal from a receiver antenna, the first signal comprising a reflected signal of a transmitted signal on the living subject, the transmitted signal being transmitted by a transmitter antenna of a wireless transmitter, the receiver antenna being adapted to be electromagnetically coupled to the transmitter antenna;
- receive a second signal from a coupling of the first received signal;
- control a self-interference cancellation circuitry for cancelling self-interferences between the first and second received signals prior to amplification;
- monitor the physiological signal characteristics from a mixing of the received second signal and the amplified attenuated received first signal.

While not explicitly described, the present embodiments may be employed in any combination or sub-combination. For example, the present principles are not limited to the described variants, and any arrangement of variants and embodiments can be used. Moreover, the present principles are not limited to the described self-interference circuitry examples and any other type of self-interference assembly is compatible with the disclosed principles. The present principles are not further limited to the described antenna coupling and are applicable to any other antenna coupling. The present principles are not further limited to the described physiological signal characteristics monitoring.

Besides, any characteristic, variant or embodiment described for a method is compatible with a sensing device and/or apparatus intended to process the disclosed method, with a computer program product comprising program code instructions and with a computer-readable storage medium storing program instructions.

### 4. BRIEF DESCRIPTION OF THE DRAWINGS

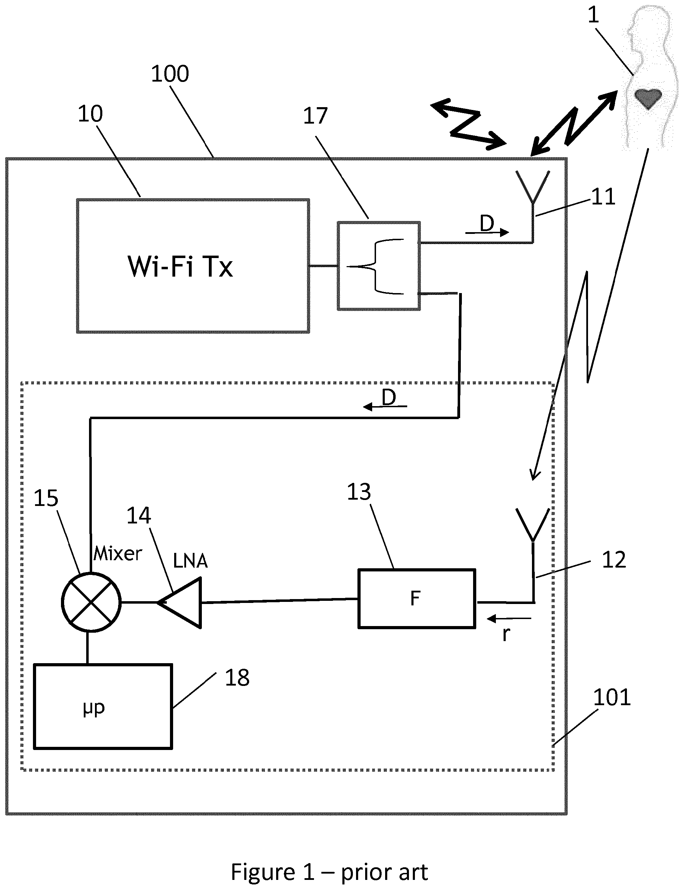

- **Figure 1** illustrates an example of a monitoring device according to a known prior art;
- **Figure 2** illustrates a monitoring system comprising a wireless sensing processing device for monitoring physiological signal characteristics of a living subject according to a specific and non-limiting embodiment of the disclosed principles;
- **Figure 3** represents an exemplary architecture of the wireless sensing processing device of figure 2 according to a specific and non-limiting embodiment of the disclosed principles;
- **Figure 4** illustrates a method for monitoring physiological signal characteristics of a living subject according to a specific and non-limiting embodiment of the disclosed principles.

### 5. DESCRIPTION OF EMBODIMENTS

Physiological signal characteristics pertaining to a moving organ such as a heart or lungs comprise statistics of the heart and/or breathing rates such as for example and without limitations an instantaneous rate, and/or average/minimum/maximum rates over a period. It further comprises alert triggers when for example a monitored (heart or breathing) rate deviates from a significant factor, or exceeds a particular value. These are only exemplary signal characteristics, and any other type of signal characteristics related to a moving organ, moving according a given frequency is compatible with the disclosed principles.

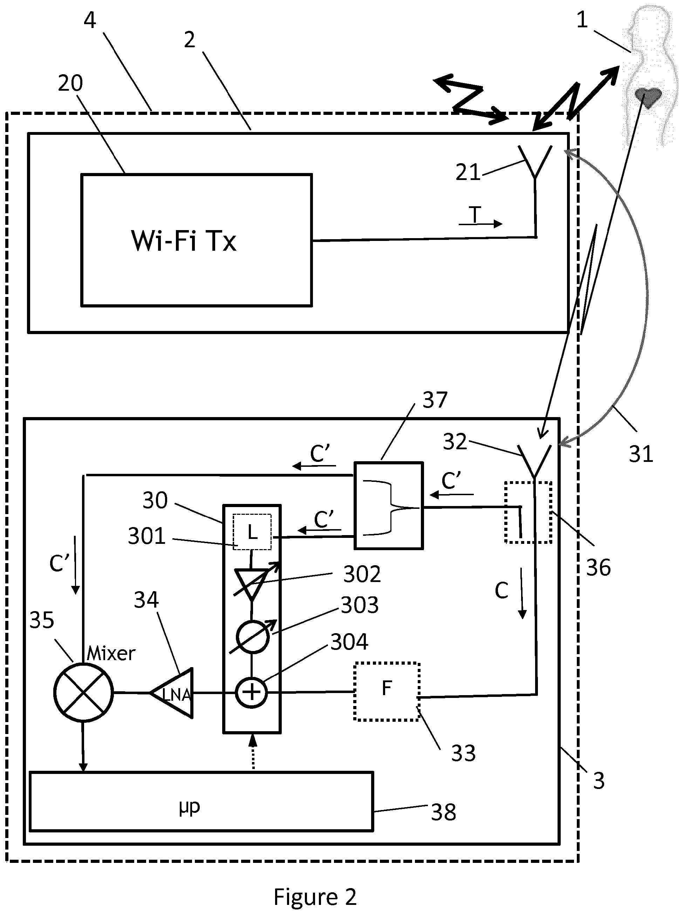

**Figure 2** illustrates a monitoring system 4 for monitoring physiological signal characteristics of a living subject 1 according to a specific and non-limiting embodiment. The monitoring system 4, comprises a wireless networking device 2 capable of transmitting a wireless signal T. The monitoring system 4 further comprises a separated wireless sensing processing device 3 also called sensing module 3, physically separable from the wireless networking device 2. The wireless networking device 2 is for example a Wi-Fi access point. But any other kind of wireless device, such as a Wi-Fi station, or any kind of wireless device, using any other kind of wireless protocol such as for example a Bluetooth device, is compatible with the disclosed principles. The wireless networking device 2 comprises a wireless (for example a Wi-Fi) transmitter 20 and a first antenna, also called transmitter antenna 21. The wireless transmitter 20 transmits a wireless signal, called transmitted signal *T.* The wireless networking device 2 is advantageously an existing piece of networking equipment in use in a given environment (for example a home environment), and the transmitted signal *T* is any wireless signal transmitted by the networking piece of equipment for its own purpose. The transmitted wireless signal *T,* contrary to the prior art, is not a dedicated diagnosis signal *D* transmitted for the sole purpose of physiological signal monitoring, but may be any wireless signal transmitted by the wireless networking device 2. According to an illustrative and non-limiting example, a user (considered as the living subject 1) is watching TV at home, the video program being transmitted to the TV over Wi-Fi by a Wi-Fi access point 2 of the user 1. The transmitted wireless signal *T* comprises in that example the transmitted video program.

The sensing module 3 comprises a second antenna 32, also called receiver antenna 32, being electromagnetically coupled to the transmitter antenna 21. In the illustrative and non-limiting example the sensing module 3 is electromagnetic coupled to the Wi-Fi access point 2 of the user 1 watching TV (not represented). To that end, for example a pre-defined slot is present on the Wi-Fi access point 2, in proximity of a transmitter antenna 21 of the Wi-Fi access point 2, and both antennas 21, 32 are electromagnetically coupled as the sensing module 3 is engaged into the pre-defined slot. The electromagnetic coupling 31 between the transmitter antenna 21 and the receiver antenna 32 results from a proximity (i.e. relatively low distance) between both antennas 21, 32 and from at least partially overlapping antenna beams. Depending on the relative position and the respective antenna directivity of both antennas 21, 31 an electromagnetic coupling 31 is created between both antennas 21, 32 so that a part noted *Cp(T)* of the transmitted signal *T* being transmitted by the transmitter antenna 21, is received by the receiver antenna 32 with a slightly attenuated amplitude and a shifted phase, wherein both the attenuation and the phase shift depend from the electromagnetic coupling 31 (the distance and the antenna directivity).

According to the specific and non-limiting embodiment, the sensing module 3 receives a first *C* and a second *C*' wireless signals from the receiver antenna 32, being electromagnetically coupled to the transmitter antenna 21. Each of the received first *C* and second *C*' wireless signals comprises a part *Cp(T)* of the transmitted signal *T* (transmitted by the transmitter antenna 21), being received by the receiver antenna 32 with an attenuated amplitude and a shifted phase due to the electromagnetic coupling. Each of the received first *C* and second *C*' wireless signals further comprises a reflected signal *r*, resulting from the modifications (i.e. reflection) of the transmitted signal *T* as it goes through the living body 1 (for example of the user watching TV at home), and reflects on various moving organs. The received first signal *C* can be noted: *C* = *Cp(T)+r.* The first signal *C* is received from the receiver antenna (32). The second signal *C*' is obtained from a coupling (36) between the sensing module and the received first signal *C*. In a first variant (not represented) the second wireless signal *C*' is obtained from a RF splitter connected to the receiver antenna 32. In an advantageous variant the second wireless signal *C*' is obtained from a RF coupler 36 connected to the receiver antenna 32, a RF coupler being less disruptive for the received signals. According to both variants, both the received first *C* and second *C*' signals share very similar characteristics and are considered nearly identical. More precisely, they contain the same information as they share the same frequency characteristics. They differ only from a phase and/or an amplitude shift.

The sensing module 3 comprises a RF splitter 37, a self-interference cancellation module 30 and a mixer 35. The RF splitter 37 is configured to split the received second signal *C*' so that the received second signal *C*' is injected in the mixer 35 and in the self-interference cancellation module 30. The received first signal *C* is filtered by an optional band pass filter 33 for attenuating unwanted frequencies, prior to be injected to the self-interference cancellation module 30. In a variant (not represented), an optional filter is inserted between the splitter 37 and the coupler 36 for attenuating unwanted frequencies.

The self-interference cancellation module 30 comprises for example a RF VGA 302 (Variable Gain Amplifier), a RF VPS 303 (Variable Phase Shifter) and a RF summer 304. In a first variant, the RF VGA and/or the RF VPS are digitally controlled, meaning that their respective gain and phase shift is controlled by configuring them according to a digital value. In a second variant, the RF VGA 302 and/or the RF VPS 303 are voltage controlled, meaning that their respective gain and phase shift is controlled according to a variable voltage being controlled, for example by the processing module 38.

In an optional variant, the self-interference cancellation module 30 comprises a limiter 301 configured to limit the received second signal *C*' prior being amplified by the VGA 302. The limiter 301 is configured to amplify and cap the received second signal *C*' for removing the reflected signal *r* being included in the received second signal *C*'. Indeed, the amplitude of the reflected signal r is much smaller than the overall signal. This is due to the fact that the greatest part of energy is received from the antenna coupling (i.e. from the transmitted signal), and a very low level of energy is received from the reflection of the signal on the moving organ. Amplifying and capping the received second signal *C*' advantageously allows to eliminate the reflected portion of the received second signal *C*'. In a second variant, the VGA 302 operates with amplitude saturation meaning that there is no amplitude variation (due to a capping) at the output of the VGA 302 eliminating the reflected signal *r* from the received second signal *C*'.

In any of the variants (with the limiter or with the VGA 302 operating with amplitude saturation), the RF VGA 302 is further configured to amplify the received second signal *C*' for reaching an amplitude close to the amplitude of the received first signal *C*. For example, both amplitudes differ from less than five percent. The RF VPS 303 is configured to shift the phase of the received second signal *C*' for reaching a phase shift close to 180° with the received first signal *C*, both signals being almost in opposition of phase. For example, both phases are in opposition within a five percent margin. The received second signal *C'*, after the amplification and the phase shift is summed with the received first signal *C* in the summer 304 or summing circuit so as to almost cancel the coupled transmitted signal from the received first signal *C*. Indeed, at the output of the summer 304, the part of the signal received from the antenna coupling is drastically attenuated, so that the reflected signal *r* (eliminated from the received second signal *C*' but still present in the received first signal *C* has been extracted and is further amplified by the LNA 34 to reach a level of amplitude within a range corresponding to proper working of the RF mixer 35. The resulting amplified signal is further mixed in a RF mixer 35 with the received second signal *C*'. In a variant (not represented) the resulting amplified signal is further mixed in a RF mixer 35 with the received first signal C. A RF mixer, as it is known to the skilled in the art multiplies two RF signals.

Assembling a RF VGA 302, a RF VPS 303 and a summer 304 as depicted in Figure 2, is an exemplary circuitry of a self-interference cancellation module 30. Any other king of circuitry adapted to cancel the coupled transmitted signal and to extract reflected signal r from the received first signal C, is compatible with the disclosed principles.

According to a first specific and non-limiting embodiment, the self-interference cancellation module 30 is statically configured, meaning that the gain of the VGA 302 and the phase shift of the VPS 303 are statically configured. The gain and the phase shift to be applied to the received second signal *C*' for cancelling the coupled part of the transmitted signal, depends on the electromagnetic coupling 36 between both antennas 21, 32. In a first variant they are determined while designing the monitoring system 4, especially while designing the location and the directivity of the transmitter antenna 21 and the receiver antenna 32. In a second variant, the gain and the phase shift to be applied to the second signal are determined by measuring the electromagnetic coupling between the antennas 21, 32 once they are in their respective position. It shall be noted that a monitoring system 4 embedding a statically configured self-interference cancellation module needs to be pre-configured before provided to a user, and does not allow the user to add a monitoring sensing module to one of its pre-existing wireless network equipment. Such monitoring system is however advantageous with regards to the prior art as it allows to deploy wireless networking equipment augmented with a physiological signal characteristics monitoring function. It further advantageously improves the flexibility of manufacturing such devices as they can be assembled from different submodules without necessarily sharing electrical interfaces.

According to a second specific and non-limiting embodiment, the self-interference cancellation module 30 is dynamically configured, meaning that the gain of the VGA 302 and the phase shift of the VPS 303 are dynamically controlled by the processing module 38. Indeed, the gain and the phase shift are controllable parameters of a looped system comprising the VGA 302, the VPS 303, the summer 304 and the processing module 38. More precisely, the gain and the phase shift are iteratively determined by the processing module 38 controlling the locked loop so as to obtain an average value of the signal at the output of the summer 304, being closed to zero. This is done by iteratively determining (i) the gain of the RF VGA 302 so that the amplified received second signal C' reaches almost a same amplitude as the received first signal *C* at the input of the summer 304 (for example within a five percent margin), and (ii) the phase of the RF VPS 303 so that the phase shifted received second signal C' is almost in opposition with the received first signal *C* at the input of the summer 304 (for example within a five percent margin). The dynamic control of the self-interference cancellation parameters based on a locked loop control is an implementation example. Any dynamic control of the self-interference cancellation module 30 so as to obtain a resulting signal at the input of the LNA 34 (i.e. at the output of the self-interference cancellation module 30) with an average level closed to zero, is compatible with the disclosed principles.

According to any of the above variant, the signal resulting from the mixing 35 of the received second signal C' with the signal resulting from the low noise amplification 34 of the output signal of the self-interference cancellation module 30, is further processed by the processing module 38. The processing module comprises one or more processor(s), which is(are), for example, a CPU, a GPU and/or a DSP (English acronym of Digital Signal Processor), along with internal memory (e.g. RAM, ROM, EPROM). The processing module further comprises one or more DAC (Digital to Analog Converter) for converting analog configuration and/or control signals of the various sensing module circuitry to digital data. The processing module 38 is configured to process the baseband signal generated by the mixer 35, from the mixing of the above-mentioned signals. The processing module 38 is configured to monitor the physiological signal characteristics based on the signal generated by the mixer 35. Monitoring the physiological signal characteristics for example includes, based on the Doppler Effect principles, extracting heart and breathing rates from the differences in frequency, phase, and/or wavelength between the mixed signals. For example, low pass filtering, and cut-off frequencies are determined for detecting envelopes of breathing and heartbeat rates. Monitoring the physiological signal characteristics further comprises, for example and without limitation, computing and logging values of physiological signal characteristics, such as average, instantaneous, max, min, ... heartbeat and/or breathing rates. Monitoring the physiological signal characteristics further comprise, for example and without limitation making available the monitored signal characteristic values over a network interface, a local or a remote display screen.

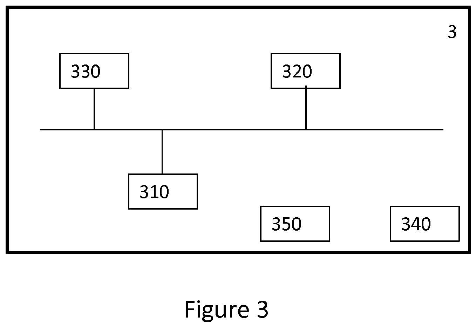

**Figure 3** represents an exemplary architecture of the wireless sensing processing device 3 according to a specific and non-limiting embodiment, where the wireless sensing processing device 3 is configured to monitor physiological signal characteristics of a living subject. The wireless sensing processing device 3 comprises one or more processor(s) 310, which is(are), for example, a CPU, a GPU and/or a DSP (English acronym of Digital Signal Processor), along with internal memory 320 (e.g. RAM, ROM, EPROM). The wireless sensing processing device 3 comprises one or several Input/Output interface(s) 330 adapted to send to display output information, such as the monitored physiological signal characteristics. In a variant, the wireless sensing processing device 3 comprises a local display mean such as a screen for displaying the monitored physiological signal characteristics. The Input/Output interface(s) 330 are further adapted to allow a user to enter commands and/or data (e.g. a keyboard, a mouse, a touchpad, a webcam, a display) so as for example to configure the wireless sensing processing device 3, and/or to send / receive data over a network interface such as for example and without limitation Ethernet, Wi-Fi, Bluetooth (any network interface being compatible with the disclosed principles). The wireless sensing processing device 3 comprises analog circuitry 350 corresponding to (and without limitation) the previously described assembly of LNA(s), RF Mixer(s), RF splitter(s), RF filter(s), RF coupler(s), antenna, self-interference cancellation and DAC (not represented). The wireless sensing processing device 3 further comprises a power source 340 which may be internal (as for example a battery) or external to the wireless sensing processing device 3.

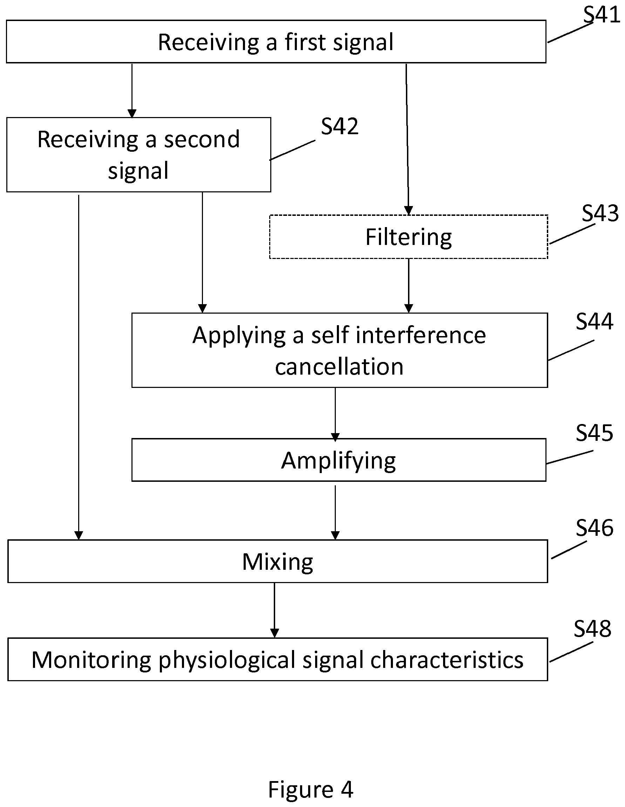

According to an exemplary and non-limiting embodiment, the wireless sensing processing device 3 further comprises a computer program stored in the memory 320. The computer program comprises instructions which, when executed by the wireless sensing processing device 3, in particular by the processor 310, make the wireless sensing processing device 3 carry out the processing method described with reference to figure 4 (see below). According to a variant, the computer program is stored externally to the wireless sensing processing device 3 on a non-transitory digital data support, e.g. on an external storage medium such as a SD Card, HDD, CD-ROM, DVD, a read-only and/or DVD drive and/or a DVD Read/Write drive, all known in the art. The wireless sensing processing device 3 thus comprises an interface to read the computer program. Further, the wireless sensing processing device 3 could access one or more Universal Serial Bus (USB)-type storage devices (e.g., "memory sticks.") through corresponding USB ports (not shown).

For the sake of clarity and without limitation, *sensing* physiological signal refers to low level signal processing such as to extract basic physiological signal characteristics, (for example the instantaneous heart/respiratory rates), while *monitoring* the physiological signal refers to higher level processing such as maintaining and/or reporting statistics of the extracted data over time. This split is given as an exemplary split and is not limitative.

According to a first exemplary and non-limiting embodiment, the monitoring system is a monitoring wireless sensing processing device 3, performing both the sensing and the monitoring functions. The processing device 3 is adapted to be electromagnetically coupled to a wireless access point. In that example, the physiological signal monitoring is performed in the processing device 3. In that example, the processing device 3 is engaged in a slot, for example pre-existing in the housing of the access point in proximity of the access point antenna so as to realize the electromagnetic coupling.

According to a second exemplary and non-limiting embodiment, the monitoring system comprises a wireless access point electromagnetically coupled to a wireless sensing processing device 3. In that second embodiment, the processing device 3 performs the sensing functions, and the access point performs the monitoring functions. In that example, both devices may be provided as a single device, or as separated but electromagnetically couplable devices. In that example, the devices (networking device and sensing device) may be further connected via a network or a bus connection so as to exchange data. In that example, the physiological signal monitoring may be performed in the access point or in the sensing device, or distributed over both devices. Any sharing of the monitoring functions between the access point and the sensing device is compatible with the disclosed principles. Moreover, any wireless networking device (different of an access point), such as wireless end device or a station is compatible with the disclosed principles.

**Figure 4** illustrates a method for monitoring physiological signal characteristics of a living subject according to a specific and non-limiting embodiment of the disclosed principles.

In the step S41, a first signal is received in a sensing module, the first signal comprising a reflected signal of a transmitted signal. The transmitted signal is transmitted by a wireless transmitter, being for example included in a wireless networking equipment, as previously described. The reflected signal provides from a reflection of the transmitted signal on the living subject. The sensing module comprises a receiver antenna, electromagnetically coupled to a transmitter antenna of the wireless transmitter. From the electromagnetic coupling between the receiver antenna and the transmitter antenna, the received first signal further comprises a coupled transmitted signal.

In the step S42, a second signal is received in the sensing module. For example the second signal is obtained from a coupling of the first signal, the coupling being performed by for example a RF coupler connected to the receiver antenna of the sensing module.

In the optional step S43, the received first signal is filtered for example in band pass filter to attenuate unwanted frequencies.

In the step S44, a self-interference cancellation is applied to the received first and second signals to cancel the coupled transmitted signal from the received first signal, so as to extract the reflected signal.

In the step S45, the extracted reflected signal, corresponding to the signal at the output of the self-inference cancellation is amplified by for example a low noise amplifier. The resulting amplified signal is mixed in the step S46 with the received second signal.

In the step S48, the mixed signals are processed so as to extract and monitor physiological signal characteristics.

## Claims

1. A method for monitoring physiological signal characteristics of a living subject (1), the method comprising:
- receiving (S41) a first signal (C) in a sensing module (3), the first signal (C) comprising a reflected signal of a transmitted signal (T) on the living subject (1), the transmitted signal (T) being transmitted by a wireless transmitter (20);
- receiving (S42) a second signal (C') in the sensing module (3) coupled to the wireless transmitter (20);
- amplifying (S45) the received first signal (C);
- mixing (S46) the received second signal (C') and the amplified received first signal to monitor (S48) the physiological signal characteristics;
the method being **characterized in that**:
- a receiver antenna (32) of the sensing module (3) is electromagnetically coupled (31) to a transmitter antenna (21) of the wireless transmitter (20);
- a self-interference cancellation (S44) is applied to the received first (C) and second (C') signals before amplifying (S45) and mixing (S46).

2. The method according to claim 1, wherein the received second signal (C') is obtained from a RF coupler (36) connected to the receiver antenna (32) of the sensing module (3).

3. The method according to any of claims 1 or 2, wherein applying a self-interference cancellation (S44) comprises applying a variable gain amplifier and a variable phase shifter to the received second signal (C'), the variable gain amplifier operating with amplitude saturation, the variable gain amplifier and the variable phase shifter being configured to extract the reflected signal from the received first signal (C).

4. The method according to any of claims 1 or 2, wherein applying a self-interference cancellation comprises limiting the received second signal (C') for removing the reflected signal, and applying a variable gain amplifier and a variable phase shifter to the received limited second signal, the variable gain amplifier and the variable phase shifter being configured to extract the reflected signal from the received first signal (C).

5. The method according to any of claims 1 to 4, wherein the variable gain amplifier and the variable phase shifter are statically configured.

6. The method according to any of claims 1 to 4, wherein the variable gain amplifier and the variable phase shifter are dynamically controlled.

7. The method according to any of claims 1 to 6, wherein the wireless transmitter (20) is a Wi-Fi access point.

8. A sensing device (3) for monitoring physiological signal characteristics of a living subject (1), the sensing device (3) comprising:
- a receiver antenna (32) for receiving a first signal (C), the first signal (C) comprising a reflected signal of a transmitted signal (T) on the living subject (1), the transmitted signal (T) being transmitted by a wireless transmitter (20);
- means for receiving a second signal (C') from a coupling to the wireless transmitter (20);
- means (34) for amplifying the received first signal (C);
- means (35) for mixing the received second signal (C') and the amplified received first signal to monitor the physiological signal characteristics;
the sensing device being **characterized in that**:
- the coupling is an electromagnetic coupling (31) between the receiver antenna (32) and a transmitter antenna (21) of the wireless transmitter (20);
- the sensing device further comprises self-interference cancellation means (30) for cancelling self-interferences between the received first (C) and second (C') signals before amplifying and mixing.

9. The sensing device according to claim 8, wherein the self-interference cancellation means (30) comprise a variable gain amplifier and a variable phase shifter, the variable gain amplifier operating with amplitude saturation, the variable gain amplifier and the variable phase shifter being configured to extract the reflected signal from the received first signal (C).

10. The sensing device according to claim 9, wherein the variable gain amplifier and the variable phase shifter are dynamically controlled.

11. An apparatus (4) for monitoring physiological signal characteristics of a living subject (1), the apparatus (4) comprising a wireless transmitter (20) adapted to transmit a transmitted signal with a transmitter antenna (21), the apparatus (4) further comprising a sensing device (3), the sensing device (3) comprising:
- a receiver antenna (32) for receiving a first signal (C), the first signal (C) comprising a reflected signal of the transmitted signal (T) on the living subject (1);
- means (36) for receiving a second signal (C') from a coupling (31) to the wireless transmitter;
- means (34) for amplifying the received first signal (C);
- means (35) for mixing the received second signal (C') and the amplified received first signal to monitor the physiological signal characteristics;
the apparatus (4) being **characterized in that**:
- the coupling being an electromagnetic coupling (31) between the receiver antenna (32) and a transmitter antenna (21) of the wireless transmitter (20);
- the sensing device further comprises self-interference cancellation means (30) for cancelling self-interferences between the received first (C) and second (C') signals before amplifying and mixing.

12. The apparatus according to claim 11, wherein the self-interference cancellation means (30) comprise a variable gain amplifier and a variable phase shifter, the variable gain amplifier operating with amplitude saturation, the variable gain amplifier and the variable phase shifter being configured to extract the reflected signal from the received first signal (C).

13. The apparatus according to claim 12, wherein the variable gain amplifier and the variable phase shifter are statically configured.

14. The apparatus according to claim 12, wherein the variable gain amplifier and a variable phase shifter are dynamically controlled.

15. A computer program product for monitoring physiological signal characteristics of a living subject, said computer program product comprising program code instructions executable by a processor for:
- receiving a first signal from a receiver antenna, the first signal comprising a reflected signal of a transmitted signal on the living subject, the transmitted signal being transmitted by a transmitter antenna of a wireless transmitter, the receiver antenna being adapted to be electromagnetically coupled to the transmitter antenna;
- receiving a second signal from a coupling of the first received signal;
- controlling a self-interference cancellation circuitry for cancelling self-interferences between the first and second received signals prior to amplification;
- monitoring the physiological signal characteristics from a mixing of the received second signal and the amplified attenuated received first signal.
